# EUROPEAN PATENT SPECIFICATION

(11) **EP 0 484 438 B1**
(45) Date of publication and mention of the grant of the patent: **10.04.1996**
(21) Application number: 90912314.3
(22) Date of filing: 27.07.1990
(51) Int. Cl.: A61K 39/04

(54) **BIOLOGICAL PREPARATION AND ITS USE**
BIOLOGISCHES PRÄPARAT UND SEINE VERWENDUNG
PREPARATION BIOLOGIQUE ET SON UTILISATION

(30) Priority: 28.07.1989 GB 8917256
(43) Date of publication of application: 13.05.1992
(73) Proprietor: UNIVERSITY COLLEGE LONDON, London WC1E 6HA (GB)
(72) Inventor: STANFORD, John, Lawson, Marden, Kent TN12 9TE (GB); ROOK, Graham, Arthur, William, Haverhill,Suffolk CB9 7EJ (GB)
(74) Representative: Woods, Geoffrey Corlett
(86) International application number: GB9001169
(87) International publication number: WO9101751

(56) References cited:
- WO-A-85/03639
- WO-A-85/05034
- DE-A- 3 728 367
- INTERNATL. JOURNAL OF LEPROSIS, vol. 56, no. 3, September 1988, US; M.G. DEO, pp. 455-463/
- REVIEWS OF INFECTIOUS DISEASES, vol. 11, no. 2, March-April 1989, The University of Chicago; G. KALLENIUS et al., pp. 349-351/

## Description

This invention relates to immunoprophylactic agents useful in immunoprophylaxis against the AIDS (acquired immune deficiency syndrome) syndrome.

DE-A-3728367 describes the use of a combination of Mycobacterium phlei cells, diisopropylammonium dichloroacetate, and ascorbic acid in the treatment of HIV infection.

WO85/03639 describes the use of antigenic material derived from Mycobacterium vaccae as an immunotherapeutic agent, in particular in the treatment of tuberculosis or leprosy.

WO85/05034 describes the use of compositions containing a mycobacterium, especially M. vaccae, in the alleviation or treatment of arthritic diseases.

The causative agent for AIDS is known to be a virus of the retrovirus family known as HIV (human immunodeficiency virus). Infection with HIV does not, however, immediately give rise to overt symptoms of the AIDS syndrome. The only indication of exposure to the virus may be the presence of antibodies thereto in the blood of an infected subject who is then described as "HIV positive". The infection may lie dormant, giving rise to no obvious symptoms, and the incubation period prior to development of the AIDS syndrome may vary from several months to decades. Development of AIDS itself may be preceded by the AIDS-related complex (ARC) which is characterised by unexplained fever, weight loss, chronic cough or diarrhoea.

The reasons for the variable period between infection with the virus and breakdown of the immune system in an infected individual are poorly understood. Factors at present unknown may trigger proliferation of the virus with consequential disruption of the immune system. The victims of the disease are then subject to various infections and malignancies which, unchecked by the disabled immune system, lead to death.

Despite the rapid growth of research into AIDS no vaccine against it is yet available: it has been suggested that the genetic variability of the virus will in fact hamper the search for an effective vaccine.

The present invention seeks to provide an immunoprophylactic agent useful in delaying or preventing onset of the AIDS syndrome, i.e. in increasing the period between infection by HIV and development of the AIDS syndrome.

The present invention provides an immunoprophylactic agent comprising antigenic and/or immunoregulatory material derived from Mycobacterium vaccae for use in immunoprophylactic treatment against the AIDS syndrome. The preparation conveniently, and therefore preferably, comprises dead cells of M. vaccae, most preferably cells which have been killed by autoclaving. The immunotherapeutic agent normally comprises more than 10⁸ microorganisms per ml of diluent, and preferably from 10⁸ to 10¹¹ killed M. vaccae microorganisms per ml of diluent. The invention includes within its scope antigenic and/or immunoregulatory material from M. vaccae for use in therapy in the prophylactic treatment of HIV infection to delay or prevent onset of the AIDS syndrome.

The diluent may be pyrogen-free saline for injection alone, or a borate buffer of pH 9.0. The diluent should be sterile. A suitable borate buffer is:

| | |
|---|---|
| Na₂B₄0₇.10H₂0 | 3.63 g |
| H₃BO₃ | 5.25 g |
| NaC1 | 6.19 g |
| Tween® | 0.0005% |
| Distilled Water | to 1 litre |

The preferred strain of M. vaccae is one denoted R877R isolated from mud samples from the Lango district of Central Uganda (J.L. Stanford and R.C. Paul, Ann. Soc. Belge Med, Trop. 1973, 53, 141-389). The strain is a stable rough variant and belongs to the aurum sub-species. It can be identified as belonging to M. vaccae by biochemical and antigenic criteria (R. Bonicke, S.E. Juhasz., Zentr albl. Bakteriol. Parasitenkd. Infection skr. Hyg. Abt. 1, Orig., 1964, 192, 133).

The strain denoted R877R has been deposited at the National Collection of Type Cultures (NCTC) Central Public Health Laboratory, Colindale Avenue, London NW9 5HT, United Kingdom on February 13th, 1984 under the number NCTC 11659.

For the preparation of the immunoprophylactic agent, the microorganism M. vaccae may be grown on a suitable solid medium. A modified Sauton's liquid medium is preferred (S.V. Boyden and E. Sorkin., J. Immunol, 1955, 75, 15) solidified with agar.

Preferably the solid medium contains 1.3% agar. The medium inoculated with the microorganisms is incubated aerobically to enable growth of the microorganisms to take place, generally at 32°C for 10 days. The organisms are harvested, then weighed and suspended in a diluent. The diluent may be unbuffered saline but is preferably borate-buffered and contains a surfactant such as Tween® 80 as described above. The suspension is diluted to give 100 mg of microorganism/ml. For further dilution, borate buffered saline is preferably used so that the suspension contains 10 mg wet weight of microorganisms/ml of diluent. The suspension may then be dispensed into 5 ml multidose vials. Although the microorganisms in the vials may be killed using irradiation e.g. from ⁶⁰Cobalt at a dose of 2.5 megarads, or by any other means, for example chemically, it is preferred to kill the microorganisms by autoclaving, for example at 10 psi for 10 minutes (115°-125°C). It has been discovered, unexpectedly, that autoclaving yields a more effective preparation than irradiation.

The immunotherapeutic agent is in general administered by injection in a volume in the range 0.1-0.2 ml, preferably 0.1 ml, given intradermally. A single dosage will generally contain from 10⁷ to 10¹⁰ killed M. vaccae microorganisms. It is preferred to administer to patients a single dose containing 10⁸ to 10⁹ killed M. vaccae. However, the dose may be repeated depending on the condition of the patient.

Although the immunotherapeutic agent will generally be administered by intradermal injection other routes, e.g. oral administration, can also be used.

Prophylactic treatment against the AIDS syndrome comprises administering to a subject, for example a subject who has been exposed to infection by HIV or a subject who has developed antibodies to HIV, antigenic and/or immunoregulatory material derived from Mycobacterium vaccae in an amount sufficient to provoke an immune response effective to delay or prevent onset of the AIDS syndrome.

For 20 to 50% of African patients with HIV infection tuberculosis is the first symptom in development of the AIDS syndrome. Tuberculosis infection is associated with significant production of interleukin 6 (IL6) and tumour necrosis factor (TNF). There is evidence to show that the addition of TNF and IL6 to HIV-infected T cells in vitro leads to increased multiplication of the virus. The TNF release associated with tuberculosis infection in an HIV-positive subject may precipitate proliferation of the HIV with consequential disruption of the function of T4 cells in the immune system and production of immunodeficiency.

It is believed that the prevention of tuberculosis or, more specifically, the inhibition of TNF, and IL6 associated (Koch) responses, will have a delaying effect on precipitation of the AIDS syndrome. The immunoprophylactic agents of the invention are believed to exert an immunomodulatory effect on pre-existent cell mediated necrotising responses, changing them to a non-necrotising form of response and that this is due to decreased production of, or a change in function of, IL6 and TNF. It is also believed that protective immunity against both tuberculosis and leprosy are enhanced.

It may be advantageous and is within the scope of the invention to use more than one strain of M. vaccae, and/or to include in the immunoprophylactic agent other mycobacterial antigens. Tuberculin may also be included.

The immunoprophylactic agent may also contain BCG (Bacillus Calmette-Guerin) vaccine, in particular the freeze-dried form of the vaccine, to promote its effect.

The immunoprophylactic agent can contain further ingredients such as adjuvants, preservatives, stabilisers etc. It may be supplied in sterile injectable liquid form or in sterile freeze-dried form which is reconstituted prior to use.

M. vaccae may be used as such or as an extract or fractionated portion of the organism to prepare immunoprophylactic agents according to the invention.

The following Example illustrates the invention.

### EXAMPLE

M. vaccae is grown on a solid medium comprising modified Sauton's medium solidified with 1.3% agar. The medium is inoculated with the microorganism and incubated for 10 days at 32°C to enable growth of the microorganism to take place. The microorganisms are then harvested and weighed and suspended in diluent to give 100 mg of microorganisms/ml of diluent. The suspension is then further diluted with buffered saline to give a suspension containing 10 mg wet weight of microorganisms/ml of diluent and dispensed into 5 ml multidose vials. The vials containing the live microorganism are then autoclaved for 10 minutes at 10 psi to kill the microorganisms and give the immunotherapeutic agent of the invention, which may (if desired) be further diluted for use.

This immunotherapeutic agent may be administered by intradermal injection in the manner already described.

## Claims (Claims for the following Contracting State(s): AT, BE, CH, DE, DK, FR, GB, IT, LI, LU, NL, SE)

1. Use of antigenic and/or immunoregulatory material derived from Mycobacterium vaccae for the manufacture of a medicament useful in delaying or preventing onset of the AIDS syndrome.

2. The use according to claim 1, wherein the antigenic and/or immunoregulatory material derived from M. vaccae comprises dead cells of M. vaccae.

3. The use according to claim 2, wherein the cells of M. vaccae have been killed by autoclaving.

4. The use according to any one of the preceding claims wherein the material derived from M. vaccae is derived from the strain as deposited at the National Collection of Type Cultures (NCTC) Central Public Health Laboratory, Colindale Avenue, London NW9 5HT, United Kingdom on February 13th, 1984 under the number NCTC 11659.

5. The use according to any one of the preceding claims wherein the material derived from M. vaccae is contained in a medicament comprising from 10⁷ to 10¹⁰ microorganisms per dose.

## Claims (Claims for the following Contracting State(s): ES)

1. Process for the preparation of an antigenic and/or immunoregulatory therapeutic agent derived from *Mycobacterium vaccae,* useful in delaying or preventing onset of the AIDS syndrome, characterized by comprising growing the microorganism *M. vaccae* on a solid medium consisting of a modified Sauton's liquid medium solidified with agar, inoculating said medium with the microorganisms, followed by incubation aerobically at about 32°C for about 10 days, to enable the growth of the microorganisms to take place; then the microorganisms are harvested, weighed and suspended in a borate-buffered diluent and containing a surfactant, in such way that the final concentrate will be in the order of 100 mg of microorganisms/ml; the thus obtained suspension is dispensed into vials of suitable size and is submitted to autoclaving at about 115°-125°C, for about 10 minutes, or otherwise to irradiation, to kill the microorganisms, thus obtaining said therapeutic agent.

2. Process according to claim 1, wherein the material derived from *M. vaccae* is derived from the strain as deposited at the national Collection of Type Cultures (NCTC) Central Public health Laboratory, United Kingdom, under the number NCTC 11659.

3. Process according to claim 1, wherein the therapeutic agent contains, per dose, antigenic and/or immunoregulatory material from 10⁷ to 10¹⁰ *M. vaccae* microorganisms.

## Patentansprüche (Patentansprüche für folgende(n) Vertragsstaat(en): AT, BE, CH, DE, DK, FR, GB, IT, LI, LU, NL, SE)

1. Verwendung von antigenem und/oder immunregulierendem vom Mycobacterium vaccae stammenden Material zur Herstellung eines bei der Verzögerung oder zur Vermeidung des Ausbruchs des AIDS-Syndroms verwendbaren Medikaments.

2. Verwendung gemäß Anspruch 1, wobei das antigene und/oder immunregulierende vom Mycobacterium vaccae stammende Material tote Zellen von M: vaccae aufweist.

3. Verwendung gemäß Anspruch 2, wobei die Zellen von M. vaccae durch Autoklavieren abgetötet worden sind.

4. Verwendung gemäß einem der vorhergehenden Ansprüche, wobei das von M. vaccae stammende Material von dem Stamm stammt, wie er bei der National Collection of Type Cultures (NCTC) Central Public Health Laboratory, Colindale Avenue, London NW9 5HT, Vereinigtes Königreich, am 13. Februar 1984 unter der Nummer NCTC 11659 hinterlegt wurde.

5. Verwendung gemäß einem der vorhergehenden Ansprüche, wobei das von M. vaccae stammende Material in einem Medikament enthalten ist, von 10⁷ bis 10¹⁰ Mokroorganismen pro Dosis aufweist.

## Patentansprüche (Patentansprüche für folgende(n) Vertragsstaat(en): ES)

1. Verfahren zur Herstellung eines antigenen und/oder immunregulierenden vom *Mycobacterium vaccae* stammenden therapeutischen Agens, das bei der Verzögerung oder zur Vermeidung des Ausbruchs des AIDS-Syndroms verwendbar ist, gekennzeichnet durch folgende Verfahrensschritte:
- Kultivieren des Mikroorganismus *M. vaccae* auf einem aus Modifiziertem Medium aus Sauton'scher Flüssigkeit, die mit Agar verfestigt wurde, bestehenden festen Medium, Impfen des Mediums mit den Mikroorganismen, gefolgt von einer Inkubation bei ungefähr 32°C für ungefähr 10 Tage, um das Wachstum der Mikroorganismen zu ermöglichen;
- anschließend werden die Mikroorganismen geerntet, gewogen und in einem boratgepufferten und eine oberflächenwirksame Substanz enthaltendem Verdünnungsmittel auf eine derartige Weise suspendiert, daß das endgültige Konzentrat in der Größenordnung von 100 mg Mikroorganismen/ml enthält;
- die so erhaltene Suspension wird auf Ampullen geeigneter Größe verteilt und zum Abtöten der Mikroorganismen einem Autoklavieren bei ungefähr 115 bis 125°C oder anderweitig einer Bestrahlung unterworfen, wodurch das therapeutische Agens erhalten wird.

2. Verfahren gemäß Anspruch 1, wobei das von *M. vaccae* stammende Material von dem Stamm stammt, wie er bei der National Collection of Type Cultures (NCTC) Central Public Health Laboratory, Vereinigtes Königreich, unter der Nummer NCTC 11659 hinterlegt wurde.

3. Verfahren gemäß Anspruch1, wobei das therapeutische Agens pro Dosis antigenes und/oder immunregulierendes Material von 10⁷ bis 10¹⁰ *M. Vaccae* Mikroorganismen enthält.

## Revendications (Revendications pour l'(les) Etat(s) contractant(s) suivant(s): AT, BE, CH, DE, DK, FR, GB, IT, LI, LU, NL, SE)

1. Utilisation de matériel antigénique et/ou immunorégulateur provenant de Mycobacterium vaccae pour la préparation d'un médicament utile pour retarder ou prévenir l'installation du syndrome du SIDA.

2. Utilisation selon la revendication 1, dans laquelle le matériel antigénique et/ou immunorégulateur provenant de M. vaccae comprend des cellules mortes de M. vaccae.

3. Utilisation selon la revendication 2, dans laquelle les cellules de M. vaccae ont été tuées par passage à l'autoclave.

4. Utilisation selon une quelconque des revendications précédentes, dans laquelle le matériel provenant de M. vaccae a pour origine la souche telle que déposée auprès de National Collection of Type Cultures (NCTC) Central Public Health Laboratory, Colindale Avenue, Londres NW9 5HT, Royaume-Uni, le 13 février 1984 sous le numéro NCTC 11659.

5. Utilisation selon une quelconque des revendications précédentes, dans laquelle le matériel provenant de M. vaccae est contenu dans un médicament comprenant de 10⁷ à 10¹⁰ micro-organismes par dose.

## Revendications (Revendications pour l'(les) Etat(s) contractant(s) suivant(s): ES)

1. Procédé de préparation d'un agent thérapeutique antigénique et/ou immunorégulateur provenant de Mycobacterium vaccae utile pour retarder ou prévenir l'installation du syndrome du SIDA, caractérisé en ce qu'il comprend la culture du micro-organisme M. vaccae sur un milieu solide constitué par un milieu liquide de Sauton modifié, solidifié par de la gélose, l'ensemencement des micro-organsimes dans ledit milieu, puis l'incubation aérobie à environ 32°C pendant environ 10 j, pour permettre la croissance des micro-organismes; on récolte ensuite les micro-organismes, on les pèse et on les met en suspension dans un diluant tamponné par du borate et contenant un tensioactif de façon que le concentré final soit de l'ordre de 100 mg de micro-organismes par ml; puis on répartit la suspension ainsi obtenue dans des flacons de taille appropriée et on la passe à l'autoclave à environ 115°-125°C pendant environ 10 min ou on la soumet à une irradiation pour tuer les micro-organismes, en obtenant ainsi ledit agent thérapeutique.

2. Procédé selon la revendication 1, dans lequel le matériel provenant de M. vaccae a pour origine la souche telle que déposée auprès de National Collection of Type Cultures (NCTC) Central Public Health Laboratory, Royaume-Uni, sous le numéro NCTC 11659.

3. Procédé selon la revendication 1, dans lequel l'agent thérapeutique contient par dose le matériel antigénique et/ou immunorégulateur de 10⁷ à 10¹⁰ micro-organismes M. vaccae.
